# EUROPEAN PATENT SPECIFICATION

(11) **EP 2 240 608 B1**
(45) Date of publication and mention of the grant of the patent: **07.06.2017**
(21) Application number: 09702857.5
(22) Date of filing: 16.01.2009
(51) Int. Cl.: C12Q 1/68

(54) **METHOD OF PRODUCING SINGLE-STRANDED DNA**
VERFAHREN ZUR HERSTELLUNG VON EINZELSTRANG-DNA
PROCÉDÉ DE PRODUCTION D'ADN MONOCATÉNAIRE

(30) Priority: 17.01.2008 FI 20085040; 12.02.2008 US 28038 P
(43) Date of publication of application: 20.10.2010
(73) Proprietor: Mobidiag Oy, 02150 Espoo (FI)
(72) Inventor: MÄKI, Minna-Mari, 00250 Helsinki (FI); PIIPARINEN, Pasi, FI-02580 Siuntio (FI); AITTAKORPI, Anne, 01670 Vantaa (FI); LINDFORS, Merja, 01450 Vantaa (FI); KIRVESKARI, Juha, 02150 Espoo (FI)
(74) Representative: Kolster Oy Ab
(86) International application number: PCT/FI2009/050038
(87) International publication number: WO 2009/090312

(56) References cited:
- EP-A- 1 479 783
- WO-A-01/94638
- WO-A-2004/046379
- MAZARS G-R ET AL: "DIRECT SEQUENCING BY THERMAL ASYMMETRIC PCR" NUCLEIC ACIDS RESEARCH, vol. 19, no. 17, 1991, page 4783, XP002532640 ISSN: 0305-1048
- ZHU LING-XIANG ET AL: "Multiplex asymmetric PCR-based oligonucleotide microarray for detection of drug resistance genes containing single mutations in Enterobacteriaceae" ANTIMICROBIAL AGENTS AND CHEMOTHERAPY, vol. 51, no. 10, October 2007 (2007-10), pages 3707-3713, XP002532643 ISSN: 0066-4804 cited in the application
- WANG ET AL: "Thermal factors influencing detection of Vibrio vulnificus using real-time PCR" JOURNAL OF MICROBIOLOGICAL METHODS, ELSEVIER, AMSTERDAM, NL, vol. 69, no. 2, 13 April 2007 (2007-04-13), pages 358-363, XP022094306 ISSN: 0167-7012
- NAGY K ZSUZSANNA ET AL: "Advantages of a New Two-Step PCR Using Degenerate Primers for Clonal Analysis of Gene-Marked Human Colony-Forming Peripheral Blood Progenitor Cells." BLOOD, vol. 100, no. 11, 16 November 2002 (2002-11-16), page Abstract No. 1712, XP009118629 & 44TH ANNUAL MEETING OF THE AMERICAN SOCIETY OF HEMATOLOGY; PHILADELPHIA, PA, USA; DECEMBER 06-10, 2002 ISSN: 0006-4971
- LAM W Y ET AL: "Rapid multiplex nested PCR for detection of respiratory viruses", JOURNAL OF CLINICAL MICROBIOLOGY, AMERICAN SOCIETY FOR MICROBIOLOGY, US, vol. 45, no. 11, 1 November 2007 (2007-11-01), pages 3631-3640, XP002489255, ISSN: 0095-1137, DOI: 10.1128/JCM.00280-07 [retrieved on 2007-09-05]

### SEQUENCE LISTING

<110> Mobidiag oy
<120> Method of producing single-stranded DNA
<130> 2062251PC
<160> 5
<170> PatentIn version 3.2
<210> 1
   <211> 20
   <212> DNA
   <213> Artificial
<220>
   <223> Primer
<220>
   <221> misc_feature
   <222> (3)..(3)
   <223> n represents inosine
<220>
   <221> misc_feature
   <222> (6)..(6)
   <223> n represents inosine
<220>
   <221> misc_feature
   <222> (9)..(9)
   <223> n represents G or T
<220>
   <221> misc_feature
   <222> (15)..(15)
   <223> n represents C or T
<220>
   <221> misc_feature
   <222> (18)..(18)
   <223> n represents A, C or T
<400> 1
   cgnccnggna tgtanatngg 20
<210> 2
   <211> 23
   <212> DNA
   <213> Artificial
<220>
   <223> Primer
<220>
   <221> misc_feature
   <222> (1)..(1)
   <223> n represents A or G
<220>
   <221> misc_feature
   <222> (2)..(2)
   <223> n represents A or C
<220>
   <221> misc_feature
   <222> (3)..(3)
   <223> n represents inosine
<220>
   <221> misc_feature
   <222> (6)..(6)
   <223> n represents A or T
<220>
   <221> misc_feature
   <222> (9)..(9)
   <223> n represents inosine
<220>
   <221> misc_feature
   <222> (12)..(12)
   <223> n represents A or G
<220>
   <221> misc_feature
   <222> (15)..(15)
   <223> n represents C or T
<220>
   <221> misc_feature
   <222> (18)..(18)
   <223> n represents inosine
<220>
   <221> misc_feature
   <222> (21)..(21)
   <223> n represents inosine
<400> 2
   nnnccnacnc cntgnagncc ncc 23
<210> 3
   <211> 24
   <212> DNA
   <213> Artificial
<220>
   <223> Primer
<400> 3
   aatacaatcg cacatacatt aata 24
<210> 4
   <211> 30
   <212> DNA
   <213> Artificial
<220>
   <223> Primer
<400> 4
   ttactcatgc catacataaa tggatagacg 30
<210> 5
   <211> 20
   <212> DNA
   <213> Artificial
<220>
   <223> Primer
<400> 5
   agacctggta tgtatattgg 20

## Description

### FIELD OF THE INVENTION

The invention relates to a method of producing single-stranded DNA molecules, and to a kit for use in said method.

### BACKGROUND OF THE INVENTION

A polymerase chain reaction (PCR) is a well-established method of amplifying nucleic acid sequences. The most commonly used PCR protocols produce a double-stranded deoxyribonucleic acid (dsDNA), in which sequences of two strands are complementary to each other. However, production of only one strand, i.e. preparation of a single-stranded DNA (ssDNA) is required in many molecular biology and biotechnology applications, including those using ssDNA as a hybridization probe.

Several approaches to produce ssDNA have been disclosed. One way is to separate the strands of the amplified dsDNA by a prolonged denaturation for example by heat or alkali. The separated strands can then be kept apart by rapid cooling. Alternatively, one strand can be selectively removed after an amplification reaction using for example exonuclease digestion where the desired strand is protected from the enzymatic activity of the exonuclease (US patent publication 5,518,900). The desired strand can also be selectively captured based on a modification of the strand, such as a biotinylated primer used in a PCR, in denaturing conditions (e.g., European patent publication EP0456304 and US patent publication 5,817,797).

ssDNA can also be created by an asymmetric PCR, in which two primers are used at different concentrations. Such a method is disclosed in US patent publication 5,066,584 where an excess of ssDNA is produced in a linear fashion after the rate-limiting primer is exhausted in exponential amplification of dsDNA. The achieved asymmetry and thus the amount of ssDNA produced are difficult to predict, especially when a mixture of competing degenerated primers are used. Another way to produce single-stranded nucleic acid molecules is to transcribe dsDNA into RNA, which can then be amplified through multiple steps into a single stranded cRNA by an isothermic Nucleic Acid Sequence Based Amplification (NASBA) method (US patent publication 5,409,818).

US patent publication 6,887,664 discloses an asynchronous thermal cycling method for producing an excess of ssDNA. The method comprises annealing two primers at two different temperatures to the first and second strand of a nucleic acid, each annealing followed by extension. A second annealing temperature is lower than a first annealing temperature. The cycle of steps can be repeated for 2 to 50 cycles or more to produce dsDNA. The steps of annealing and extending a second primer can be omitted in the last one or more cycles to produce an excess of ssDNA. When used for ssDNA production, however, the method is very time consuming. This is due to the two different annealing temperatures for dsDNA production before ssDNA production can be initiated in the last cycles.

Mazars et al., NAR, 1991, 19: 4783 discloses a method of amplifying genomic DNA in which primers having different melting temperatures (Tm) are employed at the same concentration. The cycling is performed in two rounds in a single container. The primers are specific for exons 7 and 8 of p53 and for exon 1 of KRAS. The first round of PCR leads to the production of double stranded DNA and single-stranded DNA is produced in the second round of PCR.

Zhu et al. (Antimicrob. Agents Chemother., 2007, 51: 3707-3713) discloses a two-round multiplex asymmetric PCR method for producing ssDNA. In the method, sequence-specific primers, as well as primers tagged with a universal unrelated sequence at their 5'ends (UT primers) are used. The UT primers are designed such that a Tₘ variance of at least 10 °C as compared to the sequence-specific primers is obtained. The first-round reaction produces double-stranded DNA, whereas the second-round reaction produces ssDNA as a higher annealing temperature enabling only the UT primers to anneal to the target is used. In this method, primer design is more challenging and restricted due to the universal sequence affecting and creating for example secondary structures. The universal unrelated sequence tag makes the primers relatively long and, thus, prolongs the time required for the DNA amplification and increases the synthesization costs.

Overall, many of these methods share a disadvantage of manual involvement and multiple processing steps. There is thus a need in the art for a reliable method of producing ssDNA.

### BRIEF DESCRIPTION OF THE INVENTION

The present invention provides a method of producing a single-stranded DNA by symmetric amplification of a desired DNA using at least one specific primer pair of an untagged first and untagged second primer, wherein said first primer has a melting temperature T_{mA} and said second primer has a melting temperature T_{mB}, and wherein annealing temperature areas of said first primer and said second primer are partly overlapping. In the method, said DNA amplification comprises the steps of a) performing a first PCR stage, which comprises repeating denaturation, annealing and extension steps, and b) performing a second PCR stage which consists of repeating denaturation, annealing and extension steps, wherein annealing and extension are combined and performed at a same temperature. Both said first and second primers anneal and extend a target at the annealing temperature of the first PCR stage, while only said second primer anneals and extends the target at the annealing temperature of the second PCR stage.

In one embodiment, T_{mA} is about 8°C to about 20°C lower than T_{mB}. In other embodiments, T_{mA} is about 8°C to about 16°C, about 8°C to about 12°C, or about 8°C to about 10°C lower than T_{mB}. In a further embodiment, the annealing in the first PCR stage is performed at a temperature T_{mA} ± 8°C. In a still further embodiment, the annealing in the second PCR stage is performed at a temperature T_{mB} ±14°C.

Both the first and second primers may consist of a mixture of degenerated primers, such as primers hybridizing with conserved regions of genes encoding *gyrB*/*parE* topoisomerases. In one embodiment, said first primer comprises at least one sequence depicted in SEQ ID NO: 1 and/or said second primer comprises at least one sequence depicted in SEQ ID NO: 2. Some embodiments of the present invention comprise use of labeled primers in the method.

The method according to the present invention may consist of the steps described above or it may comprise additional steps. The method may also be used for multiplex DNA amplification, wherein more than one first and more than one second primer are used for amplifying multiple target DNAs simultaneously. In other embodiments, only one first and second primer is used.

The present invention further provides a kit, as defined in the claims, for use in the present method. The kit comprises at least one specific primer pair of an untagged first primer having a melting temperature T_{mA} and an untagged second primer having a melting temperature T_{mB}, and wherein annealing temperature areas of said first primer and said second primer are partly overlapping, and wherein both said first and second primers anneal and extend a target at an annealing temperature of a first PCR stage, while only said second primer anneals and extends the target at an annealing temperature of a second PCR stage. In some embodiments, the kit comprises only one first and second primer.

Said first and second primers may each be a mixture of degenerated primers, such as primers hybridizing with conserved regions of genes encoding *gyrB*/*parE* topoisomerases. In one embodiment, said first primer comprises at least one sequence depicted in SEQ ID NO: 1 and said second primer comprises at least one sequence depicted in SEQ ID NO: 2. In some embodiments, primers provided in the kit are labeled primers.

Furthermore, one embodiment comprises said primers being contained in a separate compartment of a biochip. In another embodiment, said biochip further comprises a separate compartment for attached oligonucleotide probes.

### BRIEF DESCRIPTION OF THE DRAWINGS

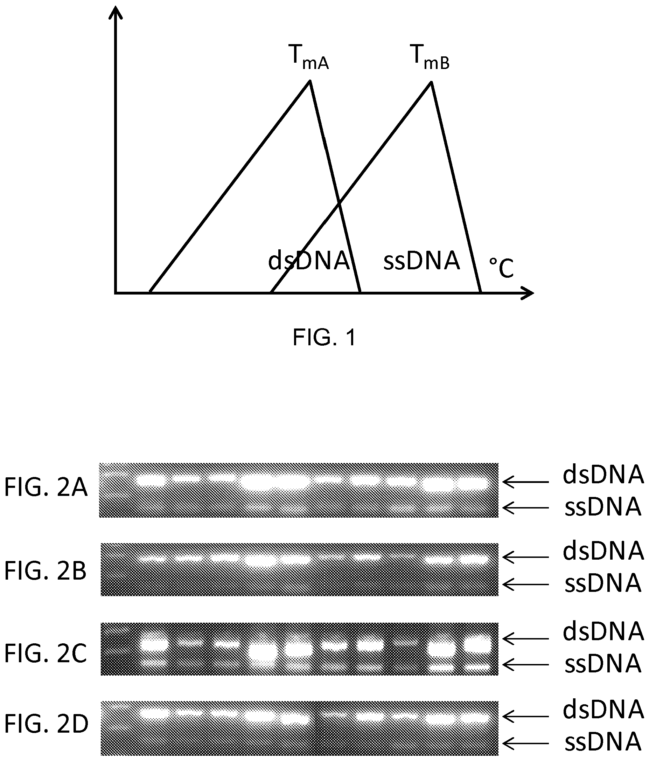

In the following, the invention will be described in greater detail by means of preferred embodiments with reference to the attached drawings, in which
Figure 1 illustrates annealing temperature areas in which a first primer and a second primer can function as a starting point for an extension in a PCR. T_{mA} represents the melting temperature of the first primer and T_{mB} represents the melting temperature of the second primer. X-axis represents an annealing temperature (°C). dsDNA is produced at an annealing temperature optimal for the first primer. The reaction is then converted to favor ssDNA production at an annealing temperature at which the first primer does not anneal to the target strand but the second primer anneals efficiently.
Figure 2 illustrates a comparison between an asymmetric PCR (Figs. 2B and 2D) and an ssDNA method of the present invention (Figs. 2A and 2C). Each lane of respective figure represents one sample, a molecular weight marker being in the first lane to the left.

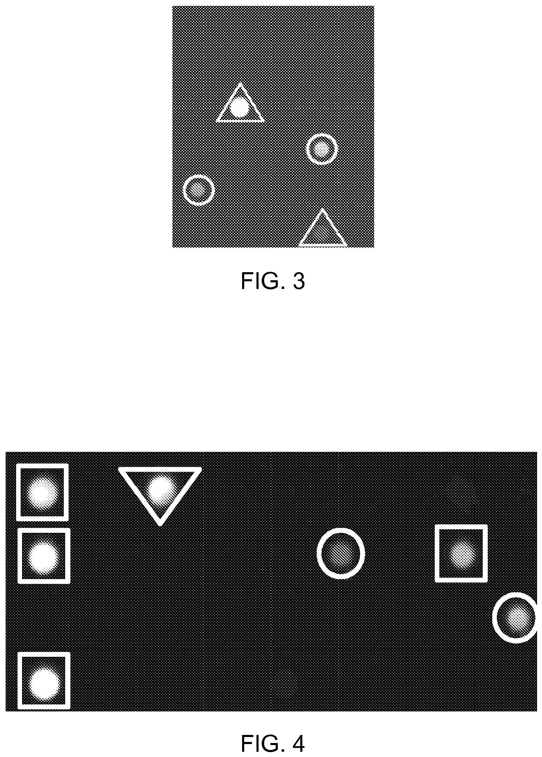

Figure 3 illustrates hybridization of a labeled ssDNA, produced by the method according to the present invention, on a microarray. A hybridized patient sample included *Pseudomonas aeruginosa* as a causative agent. The microarray included two specific *Pseudomonas aeruginosa* probes (circled spots) that bound specifically to the target strands. Two positive control oligonucleotides were also detectable (triangle-marked spots).
Figure 4 illustrates simultaneous detection of *S. aureus* (triangle-marked spot) and a *mecA* gene (circled spots) on a microarray. The *mecA* spots on the microarray comprised of two specific *mecA* oligos per spot. Four control oligonucleotides were also detected (squared spots).

### DETAILED DESCRIPTION OF THE INVENTION

The present invention relates to a novel method of producing a single-stranded DNA in a symmetric PCR amplification reaction. The method comprises two distinct PCR stages: a first stage of PCR produces dsDNA and a second stage of PCR produces ssDNA. The method does not require manual involvement thus minimizing the contamination risk and processing time. It also enables the amount of produced ssDNA to be determined. Furthermore, the whole procedure can be conveniently performed in a single PCR amplification reaction.

In this context, the term "symmetric PCR" refers to a PCR amplification wherein equal concentrations of members of a primer pair are used. The term "asymmetric PCR" refers to a PCR amplification in which members of a primer pair are used at different concentrations.

The present invention is based on untagged primers that are designed so that an annealing temperature area, in which a primer and a template DNA complex are stabile enough to allow a polymerase to extend the primer efficiently, of a first primer is overlapping but lower than the corresponding annealing temperature area of a second primer, as illustrated in Figure 1. In other words, the annealing temperature areas of said first primer and said second primer are partly non-overlapping. The first stage of the reaction is performed at an annealing temperature optimal to the first primer. In the second stage, the annealing temperature is increased close to an extension temperature at which the polymerase replicates the DNA at an optimal rate. The annealing and extension are then performed at the same temperature in a combined fashion as a two-step PCR. The annealing temperature of the second stage does not allow the first primer to anneal. Primers can be designed in such a way that either of the DNA strands can act as a template for ssDNA production.

In this context, the term "untagged" refers to primers which do not comprise any additional nucleotide sequences at their 5' or 3' end. However, such untagged primers may comprise other reactive biomolecules, such as labels, at their 5' or 3' end or incorporated into their target-specific sequence.

In this context, the term "primer" refers to an oligonucleotide, preferably single-stranded, which has an ability to initiate a DNA synthesis under appropriate conditions, usually including nucleotides and a DNA polymerase. A suitable length of the primer varies, typically ranging from 10 to 100 nucleotides. In the context of the present invention, oligonucleotide analogues, such as peptide nucleic acids (PNA), or oligonucleotides comprising modified bases, such as inosines, can act as primers and are thus included in the definition of a primer. A primer does not need to be of an exactly the same sequence as the template nucleic acid but must be sufficiently complementary to hybridize with the template. Furthermore, the term "primer" includes primer mixtures, such as mixtures of degenerated primers.

A melting temperature (Tₘ) of a primer is the temperature (under defined ionic strength and pH) at which 50% of hydrogen bonds between base pairs in a nucleic acid strand are present. Typically, an annealing step in PCR is performed approximately 5°C below Tₘ. A too low annealing temperature can cause unspecific binding and a too high annealing temperature produces only a small amount of the desired product. Tₘ (°C) can be calculated for example by the equation: 81.5 + 16.6 log [Na⁺]+0.41(%GC) - 0.61 (%for) - 500/N, in which [Na⁺] is the concentration of monovalent cations, %GC is the percentage of guanine and cytosine, %for is the percentage of formamide, and N is the length of the oligonucleotide.

An untagged primer pair for use in the present invention is designed to have a difference of about 8°C to about 20°C, preferably about 8°C to about 16°C, and more preferably about 8°C to about 12°C, and even more preferably about 8°C to about 10°C between the melting temperatures (Tₘ) of the first and the second primer. Tₘ of the first primer (T_{mA}) is lower than Tₘ of the second primer (T_{mB}). Multiple untagged primer pairs can be designed to be used simultaneously in the method of the present invention.

In the first stage of the PCR of the method according to the present invention, dsDNA is produced by a symmetric PCR according to protocols well known in the art. This reaction comprises repeating denaturation, annealing, and extension steps. Optionally, the first stage of the PCR may further comprise a touch-down PCR part in which the annealing temperature is decreased after each cycle for example by 1°C. Typically, the first stage of the PCR amplification reaction of the method comprises about 30 amplification cycles. The number of cycles can vary from about 10 to about 50.

After an appropriate number of amplification cycles in the first stage, the amplification reaction is converted to favor ssDNA production from one DNA strand, i.e. a target strand, of the template. This is achieved by using a two-step PCR with an annealing temperature high enough (about 63°C to about 73°C) to enable only the second primer to bind the target strand and to allow annealing and extension to occur in a combined fashion at the same temperature. As Tₘ of the first primer is lower than Tₘ of the second primer, the high annealing temperature used in the second stage favors annealing of the second primer and subsequent amplification of ssDNA from the target strand. In other words, the second primer has Tₘ which allows annealing within a wide temperature range, and thus, first allows the production of dsDNA at a lower annealing temperature and then the production of ssDNA at a higher annealing temperature. If necessary, it is also possible to perform an additional denaturation step after PCR.

In one embodiment of the method, the annealing temperature of the first stage can be defined as T_{mA} ±8°C and preferably as T_{mA} ±6°C, and of the second stage as T_{mB} ±148°C and preferably as T_{mB} ±12°C while still maintaining the difference of about 8°C to about 20°C between melting temperatures of the primers and conditions under which the first primer does not anneal in the second annealing temperature.

Primers can incorporate additional features into an amplified DNA which allow the DNA to be detected or immobilized but do not affect the basic property of the primer, i.e. a DNA amplification capability. A number of known labeling methods based either on labeled primer or nucleotides can be used to produce a labeled DNA for use in the present invention. Examples of suitable labeling methods include fluorescent labels (e.g., Cy5, Cy3, Cy2, Tex-asRed, FITC, Alexa 488, TMR, FluorX, ROX, TET, or HEX), radioactive labels (e.g., ³²P, ³³P, or ³³S), chemiluminescent labels (e.g., HiLight Single-Color Kit), and colorimetric detection (e.g., based on biotin labeling and detection of biotin-streptavidin-enzyme conjugate).

DNA to be amplified can be obtained from various sources and samples suspected to contain nucleic acids. These include bodily fluids, such as whole blood, saliva, sputum, urine, fecal, peritoneal and pleural fluids, tissue samples, cell or microbial cultures, environmental samples, and food or feed samples. Appropriate methods of extracting or isolating DNA from such samples are readily available in the art.

In the method of the present invention, reagents used in DNA amplification by PCR can be any reagents that are conventionally used for the amplification of DNA and are well known among those skilled in the art. Suitable and cost-effective reagents which are commercially available include different types of DNA polymerases and buffers thereof (e.g., AmpliTaq GOLD™, AmpliTaq® LD, DyNAzyme™, TaqPlus® Precision, or HotStart-Taq®), nucleotides or pre-prepared mixtures of nucleotides (e.g., Sigma, Applied Biosystems, or Amersham Biosystems), and MgCl₂ (whereby a product from the manufacturer of the DNA polymerase is generally used). Preferably, the DNA polymerase used is HotStartTaq® (Qiagen).

The equipment used for amplification can be any suitable device (e.g., Biometra® T1 Thermocycler, Applied Biosystems GenAmp® PCR system 2700, or Eppendorf Mastercycler®). Practically all devices and equipment suitable for DNA amplification can be used, and amplification can also be performed manually by transferring reaction tubes from one temperature to another. In addition, amplification can be performed directly on a biochip comprising specific wells for the PCR, and a specific hybridization area, such as a microarray, whereto the probes and control oligonucleotides can be attached.

The present method can be used to produce ssDNA for various purposes, such as hybridization-based assays of different type, or sequencing. For example, in addition to producing labeled targets for microarrays, longer microarray probes (>75 to 100 nucleotides), which cannot be optimally synthesized by current oligonucleotide synthesis methods, could be produced by the method of the present invention.

Furthermore, the present invention provides a kit, as defined in the claims, for use in the method according to the present invention. The kit comprises an untagged first and an untagged second primer described in more detail above, wherein said first primer has a melting temperature T_{mA} and said second primer has a melting temperature T_{mB}, and wherein annealing temperature areas of said first primer and said second primer are partly overlapping. The kit may further comprise some or all necessary reagents, such as buffers, nucleotides, controls, MgCl₂, and a polymerase for amplification reaction. Furthermore, the kit may comprise one or more additional primers thus being suitable for multiplex PCR.

In one embodiment, the kit may further comprise a device wherein DNA amplification is performed. Such devices include incubators, thermal cyclers and biochips of different type.

### Example 1. Amplification of single-stranded DNA

DNA from a clinical patient sample suspected to have a bacterial infection was extracted using a nucleic acid extraction robot (Nuclisens® easyMAG™, bioMérieux, France). After DNA extraction, the desired target was amplified by PCR. First, a reaction mixture containing 1xHot Start Taq® PCR buffer (Qiagen, Germany), in which MgCl₂ was added so that the final concentration was 2.0 mM, 300 µM of each of dATP, dGTP, dCTP, and dTTP (Finnzymes, Finland), 1.5 g/l BSA (EuroClone, Italy), and 0.125 U/µl Hot Start Taq® DNA polymerase (Qiagen, Germany) was prepared. Broad-range primer mixtures (Table 1) which originate from conserved regions of genes encoding topoisomerase genes *gyrB* and *parE* and which efficiently amplify DNA of different bacteria, were added to the mixture so that the final concentration of both gB3F and Cy5-labeled gB4R primer mixtures (ordered from Thermo Electron, USA) was 1 µM. Finally, 1.5 µl of an isolated DNA was added and a total volume was brought to 15 µl.

**Table 1. Primers used in amplification reaction**

| Name of primer | Amplified gene | Tₘ(°C) Mean | Sequence 5'->3' |
|---|---|---|---|
| gB3F | *gyrB* | 57.8 | CGICCIGGKATGTAYATHGG (SEQ ID NO:1) |
| Cy5-gB4R | *gyrB* | 69.0 | RMICCWACICCRTGYAGICCICC (SEQ ID NO:2) |

In the primer sequences I represents a base inosine; K represents base G or T; Y represents base C or T; H represents base A or C or T; R represents base A or G; M represents base A or C; and W represents base A or T.

In Tₘ calculations, an analysis program provided by Integrated DNA Technologies was applied using Na⁺ concentration of 50 mM and oligo concentration of 0.25 µM. Primers were designed so that a Cy5-gB4R primer had higher Tₘ and thus produced ssDNA in the reaction. The difference between mean Tₘ of the primers was 11.2°C.

The PCR was performed using a thermal cycler (Eppendorf, Germany). The first stage of the PCR was started by a 15-minute denaturation step at 95°C. This was followed by 36 cycles of 10 sec at 96°C, 20 sec at 52°C, and 5 sec at 72°C to produce dsDNA. Next, the second stage of the PCR was performed by using 10 cycles of 10 sec at 95°C and 30 sec at 67°C. The amplification products were analyzed by agarose gel electrophoresis using SYBR® Green II (Invitrogen, USA) to demonstrate the amplification of bacterial ssDNA.

### Example 2. Comparison between method of the present invention and asymmetric PCR

An objective of this experiment was to compare the method of the present invention with an asymmetric PCR.

A bacterial DNA was isolated from different bacterial culture isolates, containing *Staphylococcus aureus, S. epidermidis, S. haemolyticus, S. lugdunesis, Entrococcus faecium, E. faecalis, Streptococcus agalactiae, Streptococcus pyogenes, Streptococcus penumoniae,* and *Lisferia monocytogenes* as causative agents, using methods described in Example 1. To an asymmetric PCR (principle described in US patent publication 5,066,584), the following reaction mixture was applied: either 0.15 µM or 0.495 µM of gB3F primer mixture (Thermo Electron, USA), either 0.6 µM or 1.98 µM of Cy5-labeled gB4R primer mixture (Thermo Electron, USA), 1xHot Start Taq® PCR buffer (Qiagen, Germany), in which MgCl₂ was added so that the final concentration was 2.0 mM, 300 µM of each of dATP, dGTP, dCTP, and dTTP (Finnzymes, Finland), 1.5 g/l BSA (EuroClone, Italy), 0.125 U/µl Hot Start Taq® DNA polymerase (Qiagen, Germany), and 1.5 µl isolated DNA in a total volume of 15 µl. The PCR was performed using the thermal cycler (Eppendorf, Germany). The following PCR program was used: a 15-minute denaturation step at 95°C, 36 cycles of 20 sec at 95°C, 35 sec at 52°C, and 20 sec at 72°C.

To the method of the present invention, the following reaction mixture was applied: either 0.6 µM or 1.98 µM of gB3F primer mixture (Thermo Electron, USA), either 0.6 µM or 1.98 µM of Cy5-labeled gB4R primer mixture (Thermo Electron, USA), 1xHot Start Taq® PCR buffer (Qiagen, Germany), in which MgCl2 was added so that the final concentration was 2.0 mM, 300 µM of each of dATP, dGTP, dCTP, and dTTP (Finnzymes, Finland), 1.5 g/l BSA (EuroClone, Italy), 0.125 U/µl Hot Start Taq® DNA polymerase (Qiagen, Germany), and 1.5 µl isolated DNA in a total volume of 15 µl. The PCR was performed using the thermal cycler (Eppendorf, Germany). A PCR program was started by a 15-minute denaturation step at 95°C. This was followed by 36 cycles of 20 sec at 95°C, 35 sec at 52°C, and 20 sec at 72°C to produce dsDNA (first stage of the PCR). Next, 10 cycles of 10 sec at 95°C and 30 sec at 67°C, and finally 5 cycles of 10 sec at 95°C and 30 sec at 69°C produced ssDNA (second stage of the PCR).

After the PCR, the success of amplification of ssDNA was verified by gel electrophoresis using an agarose gel containing SYBR® Green II (Invitrogen, USA). Figure 2 demonstrates that in both low (Figs. 2A and 2B) as well as high (Figs. 2C and 2D) primer concentrations the production of ssDNA as well as dsDNA is more effective by the method of the present invention (Figs. 2A and 2C) than by an asymmetric PCR (Figs. 2B and 2D).

### Example 3. Hybridization of labeled single-stranded DNA on microarray

DNA, extracted from a positive blood culture sample of a sepsis patient, was amplified as described in Example 2 using 1 µM primer mixtures. The hybridization of the amplified DNA was performed on a biochip containing a specific hybridization area, a microarray, whereto probes specific for different target bacteria were attached. Useful species-specific probes are disclosed for example in International patent publication WO2004046379.

The hybridization reaction mixture contained about 50 to 100 ng of labelled target, 2xhybridization buffer (consisting of 1 tablet PBS pH 7.4 (Sigma, USA), 0.7 M NaCl (Fluka, Switzerland), 0.1% Tween® 20 (100%, Fluka, Switzerland), 2xDenhardt's solution (5x, Sigma, USA), 20 µg/ml salmon sperm DNA (dsDNA) (Amersham, United Kingdom)), hybridization control oligonucleotides, and sterile water. The volume of the reaction mixture was 25 µl. The 2xhybridization buffer was pre-warmed to 55°C before use. The hybridization reaction mixture was transferred to a hybridization area of the biochip. The hybridization area was sealed with clamps and the biochip was placed in a special hybridization device. During 10 min hybridization at 55°C, a Cy5-labeled ssDNA target bound to the complementary probe sequence immobilized on the microarray, creating a labeled double-stranded structure.

After the hybridization step, the biochip was briefly washed in order to remove a non-hybridized DNA. The washing steps were carried out as follows: in 2xSSC solution for 1 min at 40°C, and in 0.2xSSC solution for 1 min at 40°C. After the washing, the biochip was dried. The biochip was analyzed with an optical reader, which consists of a LED light source and a CCD camera. The detection is based on a fluorescent signal released by the Cy5-labeled ssDNA and captured by the camera.

An example of the hybridization result is presented in Figure *3**. Pseudomonas aeruginosa* was detected in the sample. The identification was in line with the result from the blood culturing. Thus, the ssDNA produced by the method of the present invention is clearly suitable for molecular biology applications.

### Example 4. Detecting staphylococcus aureus and mecA gene

DNA extracted from a patient derived bacterial culture isolate of methicillin resistant *Staphylococcus aureus* (MRSA) was amplified using the method described below.

DNA was isolated from the sample to be analyzed using a nucleic acid extraction robot (Nuclisens® easyMAG™, bioMérieux, France). After DNA isolation, the desired target was amplified using PCR. First, a reaction solution was prepared. The PCR reaction mixture contained 1 µM of gB3F primer mixture (SEQ ID NO: 1; ordered from Thermo Electron, USA), 1 µM of Cy5-labeled gB4R primer mixture (SEQ ID NO: 2; ordered from Thermo Electron, USA), 0.25 µM *mecA* forward primer (SEQ ID NO: 3), 0.25 µM Cy5-labeled *mecA* reverse primer (SEQ ID NO: 4), 0.165 µM of *S. aureus* specific topoisomerase primer (SEQ ID NO: 5), 1xHot Start Taq® PCR buffer (Qiagen, Germany), in which MgCl₂ was added so that the final concentration was 2.0 mM, 300 µM of each of dATP, dGTP, dCTP, and dTTP (Finnzymes, Finland), 1.5 g/l BSA (EuroClone, Italy), 0.125 U/µl Hot Start Taq® DNA polymerase (Qiagen, Germany), and 1.5 µl isolated DNA in a total volume of 15 µl.

The Tₘs of gB3F and Cy5-gB4R primer mixtures are presented above in Example 1. The *mecA* forward primer (SEQ ID NO: 3) was designed to have a Tₘ of 48.5 °C while the *Cy*5*-mecA* reverse primer (SEQ ID NO:4) was designed to have a Tₘ of 56.5 °C. The difference between the Tₘs of the *mecA* primers was 8°C.

The PCR was performed using a thermal cycler (Mastercy-cler®, Eppendorf, Germany). The following PCR program was used: a 15 min denaturation step at 95°C, 36 cycles of 20 sec at 95°C, 35 sec at 52°C, 20 sec at 72°C, after which 10 cycles of 10 sec at 95°C, 30 sec at 67°C, and finally 5 cycles of 10 sec at 95°C, 30 sec at 69°C. After the PCR, the success of amplification of DNA was verified by gel electrophoresis using a 2% agarose gel containing SYBR® Green II (Invitrogen, USA).

*S. aureus*-specific toposiomerase probes and *mecA*-specific probes were attached to a silicon based biochip, and the amplified target ssDNAs were hybridized therewith. The hybridization reaction mixture contained around 50 to 100 ng of labeled target, 2xhybridization buffer (consisting of 1 tablet PBS pH 7.4 (Sigma, USA), 0.7 M NaCl (Fluka, Switzerland), 0.1% Tween® 20 (100%, Fluka, Switzeriand), 2xDenhardt's (5x, Sigma, USA), 20 µg/ml salmon sperm DNA (dsDNA) (Amersham, United Kingdom)), hybridization control oligonucleotides, and sterile water. The volume of the reaction mixture was 25 µl. The 2xhybridization buffer was pre-warmed to 55°C before use. The hybridization reaction mixture was transferred to a hybridization area of the biochip. The hybridization area was sealed with clamps and the biochip was placed in a special hybridization device. During 10 min hybridization at 55°C, a Cy5-labeled ssDNA target bound to the complementary probe sequence immobilized on the microarray creating a labeled double-stranded structure.

After the hybridization step, the biochip was briefly washed in order to remove non-hybridized DNA. The washing steps were carried out as follows: in 2xSSC solution for 2.5 min at 40°C, and in 0.2xSSC solution for 2.5 min at 40°C. After the washing, the biochip was dried. The biochip was analyzed with an optical reader, which consists of a LED light source and a CCD camera. The detection is based on a fluorescent signal released by the Cy5-labeled gene product and captured by the camera. An example of the hybridization result, illustrating the detection of an *S. aureus* and *mecA*-gene, is presented in Figure 4. No hybridization was observed with a *Staphylococcus* genus-specific probe, implying that the patient had an MRSA infection. The identification was in line with the result from blood culturing. Thus, the results demonstrate efficient and specific production of ssDNA by the method according to the present invention.

It will be obvious to a person skilled in the art that as technology advances, the inventive concept can be implemented in various ways. The invention and its embodiments are not limited to the examples described above but may vary within the scope of the claims.

## Claims

1. A method of producing a single-stranded DNA by symmetric amplification of a desired DNA using at least one specific primer pair of an untagged first and untagged second primer, said amplification comprising the steps of:
a) performing a first PCR stage which comprises repeating steps of denaturation, annealing of both said first and second primers, and extension thereof,
b) performing a second PCR stage which consists of repeating denaturation, annealing and extension steps, wherein annealing and extension are combined and performed at one temperature in which only said second primer anneals and extends the target DNA,
wherein said first primer has a melting temperature T_{mA} and said second primer has a melting temperature T_{mB}, and wherein annealing temperature areas of said first primer and said second primer are partly overlapping, and
wherein the definition "untagged" refers to primers which do not comprise any additional nucleotide sequences at their 5' or 3' end.

2. The method according to claim 1, wherein T_{mA} is about 8°C to about 20°C lower than T_{mB}.

3. The method according to claim 1, wherein in step a) said annealing is performed at a temperature T_{mA} ± 8°C.

4. The method according to claim 1, wherein in step b) said annealing is performed at a temperature T_{mB} ±14°C.

5. The method according to any preceding claim, wherein both the first and the second primer consists of a mixture of degenerated primers.

6. The method according to any preceding claim, wherein said first and second primers hybridize with conserved regions of genes encoding *gyrB* and *parE* topoisomerases.

7. The method according to claim 6, wherein said first primer comprises a sequence depicted in SEQ ID NO: 1 and said second primer comprises a sequence depicted in SEQ ID NO: 2; or said first primer comprises a sequence depicted in SEQ ID NO: 3 and said second primer comprises a sequence depicted in SEQ ID NO: 4.

8. The method according to any preceding claim, wherein said primers are labeled.

9. A kit for producing a single-stranded DNA by the method according to claim 1, comprising at least one specific primer pair of an untagged first primer having a melting temperature T_{mA} and an untagged second primer having a melting temperature T_{mB}, and
wherein annealing temperature areas of said first primer and said second primer are partly overlapping, and wherein both said first and second primers anneal and extend a target at an annealing temperature of a first PCR stage, while only said second primer anneals and extends the target at an annealing temperature of a second PCR stage, and
wherein the definition "untagged" refers to primers which do not comprise any additional nucleotide sequences at their 5' or 3' end, and
wherein said first primer of the at least one primer pair comprises a sequence depicted in SEQ ID NO: 1 and said second primer of the at least one primer pair comprises a sequence depicted in SEQ ID NO: 2; or
said first primer of the at least one primer pair comprises a sequence depicted in SEQ ID NO: 3 and said second primer of the at least one primer pair comprises a sequence depicted in SEQ ID NO: 4..

10. The kit according to claim 9, wherein T_{mA} is about 8°C to about 20°C lower than T_{mB}.

11. The kit according to claim 9, wherein T_{mA} is about 8°C to about 10°C lower than T_{mB}.

12. The kit according to any one of claims 9 to 11, wherein both the first and the second primer consist of a mixture of primers.

13. The kit according to any one of claims 9 to 12, wherein said first and second primers hybridize with conserved regions of genes encoding *gyrB* and *parE* topoisomerases.

14. The kit according to any one of claims 9 to 13, wherein said primers are contained in a separate compartment of a biochip.

15. The kit according to claim 14, wherein said biochip further comprises a separate compartment for attached oligonucleotide probes.

16. Use of a kit for producing a single-stranded DNA by the method according to any one of claims 1 to 8, wherein said kit comprises at least one specific primer pair of an untagged first primer having a melting temperature T_{mA} and an untagged second primer having a melting temperature T_{mB}, and
wherein annealing temperature areas of said first primer and said second primer are partly overlapping, and wherein both said first and second primers anneal and extend a target at an annealing temperature of a first PCR stage, while only said second primer anneals and extends the target at an annealing temperature of a second PCR stage, and
wherein the definition "untagged" refers to primers which do not comprise any additional nucleotide sequences at their 5' or 3' end.

## Patentansprüche

1. Verfahren zur Herstellung einer einsträngigen DNA durch symmetrische Amplifikation einer gewünschten DNA mithilfe von wenigstens einem spezifischen Primerpaar aus einem unmarkierten ersten und einem unmarkierten zweiten Primer, wobei die Amplifikation die folgenden Schritte umfasst:
a) Durchführen einer ersten PCR-Stufe, die das Wiederholen der Schritte des Denaturierens und Anlagerns sowohl des ersten als auch des zweiten Primers und deren Verlängerung umfasst,
b) Durchführen einer zweiten PCR-Stufe, die aus dem Wiederholen der Schritte des Denaturierens, Anlagerns und Verlängerns besteht, wobei das Anlagern und Verlängern kombiniert werden und bei einer Temperatur durchgeführt werden, bei der sich nur der zweite Primer anlagert und die Ziel-DNA verlängert,
wobei der erste Primer eine Schmelztemperatur T_{mA} aufweist und der zweite Primer eine Schmelztemperatur T_{mB} aufweist, und wobei die Anlagerungstemperaturbereiche des ersten Primers und des zweiten Primers teilweise überlappend sind, und
wobei sich die Definition "unmarkiert" auf Primer bezieht, die keine zusätzlichen Nukleotidsequenzen an ihrem 5'- oder 3'-Ende umfassen.

2. Verfahren nach Anspruch 1, wobei T_{mA} etwa 8 °C bis etwa 20 °C niedriger als T_{mB} ist.

3. Verfahren nach Anspruch 1, wobei im Schritt a) die Anlagerung bei einer Temperatur von T_{mA} ±8 °C durchgeführt wird.

4. Verfahren nach Anspruch 1, wobei im Schritt b) die Anlagerung bei einer Temperatur von TmB ±14 °C durchgeführt wird.

5. Verfahren nach einem der vorhergehenden Ansprüche, wobei sowohl der erste als auch der zweite Primer aus einer Mischung aus degenerierten Primern besteht.

6. Verfahren nach einem der vorhergehenden Ansprüche, wobei der erste und der zweite Primer mit konservierten Regionen von Genen hybridisieren, die *gyrB-* und *parE*-Topoisomerasen codieren.

7. Verfahren nach Anspruch 6, wobei der erste Primer eine in SEQ ID NO: 1 abgebildete Sequenz umfasst und der zweite Primer eine in SEQ ID NO: 2 abgebildete Sequenz umfasst; oder wobei der erste Primer eine in SEQ ID NO: 3 abgebildete Sequenz umfasst und der zweite Primer eine in SEQ ID NO: 4 abgebildete Sequenz umfasst.

8. Verfahren nach einem vorhergehenden Anspruch, wobei die Primer mit einer Kennzeichnung versehen sind.

9. Kit zur Herstellung einer einsträngigen DNA durch das Verfahren nach Anspruch 1, umfassend wenigstens ein spezifisches Primerpaar aus einem unmarkierten ersten Primer mit einer Schmelztemperatur T_{mA} und einem unmarkierten zweiten Primer mit einer Schmelztemperatur T_{mB}, und
wobei Anlagerungstemperaturbereiche des ersten Primers und des zweiten Primers teilweise überlappend sind und wobei sowohl der erste als auch der zweite Primer bei einer Anlagerungstemperatur einer ersten PCR-Stufe an ein Ziel anlagern und dieses verlängern, während nur der zweite Primer bei einer Anlagerungstemperatur einer zweiten PCR-Stufe an das Ziel anlagert und dieses verlängert, und
wobei sich die Definition "unmarkiert" auf Primer bezieht, die keine zusätzlichen Nukleotidsequenzen an ihrem 5'- oder 3'-Ende umfassen,
wobei der erste Primer des wenigstens einen Primerpaars eine Sequenz umfasst, die in SEQ ID NO: 1 abgebildet ist, und wobei der zweite Primer des wenigstens einen Primerpaars eine Sequenz umfasst, die in SEQ ID NO:2 abgebildet ist; oder wobei der erste Primer des wenigstens einen Primerpaars eine Sequenz umfasst, die in SEQ ID NO: 3 abgebildet ist, und wobei der zweite Primer des wenigstens einen Primerpaars eine Sequenz umfasst, die in SEQ ID NO:4 abgebildet ist.

10. Kit nach Anspruch 9, wobei T_{mA} etwa 8 °C bis etwa 20 °C niedriger als T_{mB} ist.

11. Kit nach Anspruch 9, wobei T_{mA} etwa 8 °C bis etwa 10 °C niedriger als T_{mB} ist.

12. Kit nach einem der Ansprüche 9 bis 11, wobei sowohl der erste als auch der zweite Primer aus einer Mischung aus Primern besteht.

13. Kit nach einem der Ansprüche 9 bis 12, wobei der erste und der zweite Primer mit konservierten Regionen von Genen hybridisieren, die *gyrB-* und *parE-*Topoisomerasen codieren.

14. Kit nach einem der Ansprüche 9 bis 13, wobei die Primer in einem separaten Kompartiment eines Biochips enthalten sind.

15. Kit nach Anspruch 14, wobei der Biochip ferner ein separates Kompartiment für verknüpfte Oligonukleotid-Sonden umfasst.

16. Verwendung eines Kits zur Herstellung einer einsträngigen DNA durch das Verfahren nach einem der Ansprüche 1 bis 8, wobei das Kit wenigstens ein spezifisches Primerpaar aus einem unmarkierten ersten Primer mit einer Schmelztemperatur T_{mA} und einem unmarkierten zweiten Primer mit einer Schmelztemperatur T_{mB} umfasst, und
wobei Anlagerungstemperaturbereiche des ersten Primers und des zweiten Primers teilweise überlappend sind und wobei sowohl der erste als auch der zweite Primer bei einer Anlagerungstemperatur einer ersten PCR-Stufe an ein Ziel anlagern und dieses verlängern, während nur der zweite Primer bei einer Anlagerungstemperatur einer zweiten PCR-Stufe an das Ziel anlagert und dieses verlängert, und
wobei sich die Definition "unmarkiert" auf Primer bezieht, die keine zusätzlichen Nukleotidsequenzen an ihrem 5'- oder 3'-Ende umfassen.

## Revendications

1. Procédé de production d'un ADN monobrin par amplification symétrique d'un ADN souhaité en utilisant au moins une paire d'amorces spécifiques d'une première amorce non marquée et d'une seconde amorce non marquée, ladite amplification comprenant les étapes consistant à :
a) effectuer un premier stade de PCR qui comprend la répétition d'étapes de dénaturation, d'annelage desdites deux première et seconde amorces, et de leur extension,
b) réaliser un second stade de PCR qui est constitué de la répétition des étapes de dénaturation, d'annelage et d'extension, dans lequel l'annelage et l'extension sont combinés et effectués à une température à laquelle seule ladite seconde amorce annelle et étend l'ADN cible,
dans lequel ladite première amorce a une température de fusion T_{mA} et ladite seconde amorce a une température de fusion T_{mB}, et dans lequel des zones de température d'annelage de ladite première amorce et de ladite seconde amorce se chevauchent en partie, et
dans lequel la définition « non marquée » se réfère à des amorces qui ne comprennent pas de séquences nucléotidiques quelconques à leur extrémité 5' ou 3'.

2. Procédé selon la revendication 1, dans lequel T_{mA} est d'environ 8 °C à environ 20 °C inférieure à T_{mB}.

3. Procédé selon la revendication 1, dans lequel, à l'étape a), ledit annelage est effectué à une température T_{mA} ± 8 °C.

4. Procédé selon la revendication 1, dans lequel, à l'étape b), ledit annelage est effectué à une température T_{mB} ± 14 °C.

5. Procédé selon l'une quelconque des revendications précédentes, dans lequel à la fois la première et la seconde amorce sont constituées d'un mélange d'amorces dégénérées.

6. Procédé selon l'une quelconque des revendications précédentes, dans lequel ladite première et ladite seconde amorce s'hybrident avec des régions conservées de gènes codant pour des topoisomérases *gyrB* et *parE.*

7. Procédé selon la revendication 6, dans lequel ladite première amorce comprend une séquence décrite dans la SEQ ID n° 1 et ladite seconde amorce comprend une séquence décrite dans la SEQ ID n° 2 ; ou ladite première amorce comprend une séquence décrite dans la SEQ ID n° 3 et ladite seconde amorce comprend une séquence décrite dans la SEQ ID n° 4.

8. Procédé selon l'une quelconque des revendications précédentes, dans lequel lesdites amorces sont marquées.

9. Kit de production d'un ADN monobrin par le procédé selon la revendication 1, comprenant au moins une paire d'amorces spécifiques formée d'une première amorce non marquée ayant une température de fusion T_{mA} et d'une seconde amorce non marquée ayant une température de fusion T_{mB}, et
dans lequel des zones de température d'annelage de ladite première amorce et de ladite seconde amorce se chevauchent en partie, et dans lequel à la fois ladite première et ladite seconde amorce annellent et étendent une cible à une température d'annelage d'un premier stade de PCR, tandis que seule ladite seconde amorce annelle et étend la cible à une température d'annelage d'un second stade de PCR, et
dans lequel la définition « non marquée » se réfère à des amorces qui ne comprennent pas de séquences nucléotidiques supplémentaires quelconques à leur extrémité 5' ou 3', et
dans lequel ladite première amorce de la au moins une paire d'amorces comprend une séquence décrite dans la SEQ ID n° 1 et ladite seconde amorce de la au moins une paire d'amorces comprend une séquence décrite dans la SEQ ID n° 2 ; ou
ladite première amorce de la au moins une paire d'amorces comprend une séquence décrite dans la SEQ ID n° 3 et ladite seconde amorce de la au moins une paire d'amorces comprend une séquence décrite dans la SEQ ID n° 4.

10. Kit selon la revendication 9, dans lequel T_{mA} est d'environ 8 °C à environ 20 °C inférieure à T_{mB}.

11. Kit selon la revendication 9, dans lequel T_{mA} est d'environ 8 °C à environ 10 °C inférieure à T_{mB}.

12. Kit selon l'une quelconque des revendications 9 à 11, dans lequel à la fois la première et la seconde amorce sont constituées d'un mélange d'amorces.

13. Kit selon l'une quelconque des revendications 9 à 12, dans lequel ladite première et ladite seconde amorce s'hybrident avec des régions conservées de gènes codant pour des topoisomérases *gyrB* et *parE.*

14. Kit selon l'une quelconque des revendications 9 à 13, dans lequel lesdites amorces sont contenues dans un compartiment séparé d'une biopuce.

15. Kit selon la revendication 14, dans lequel ladite biopuce comprend en outre un compartiment séparé pour des sondes oligonucléotidiques fixées.

16. Utilisation d'un kit pour la production d'un ADN monobrin par le procédé selon l'une quelconque des revendications 1 à 8, dans laquelle ledit kit comprend au moins une paire d'amorces spécifiques formée d'une première amorce non marquée ayant une température de fusion T_{mA} et d'une seconde amorce non marquée ayant une température de fusion T_{mB}, et
dans laquelle des zones de température d'annelage de ladite première amorce et de ladite seconde amorce se chevauchent en partie, et dans laquelle à la fois ladite première et ladite seconde amorce annellent et étendent une cible à une température d'annelage d'un premier stade de PCR, tandis que seule ladite seconde amorce annelle et étend la cible à une température d'annelage d'un second stade de PCR, et
dans laquelle la définition « non marquée » se réfère à des amorces qui ne comprennent pas de séquences nucléotidiques supplémentaires quelconques à leur extrémité 5' ou 3'.
